# EUROPÄISCHE PATENTSCHRIFT

(11) **EP 3 747 090 B1**
(45) Veröffentlichungstag und Bekanntmachung des Hinweises auf die Patenterteilung: **13.03.2024**
(21) Anmeldenummer: 19701174.5
(22) Anmeldetag: 16.01.2019
(51) Int. Cl.: H01S 3/11, A61F 9/008

(54) **VERFAHREN ZUM AUSSENDEN VON LASERLICHT**
METHOD FOR EMITTING LASER LIGHT
PROCÉDÉ D'ÉMISSION DE LUMIÈRE LASER

(30) Priorität: 01.02.2018 DE 102018201508
(43) Veröffentlichungstag der Anmeldung: 09.12.2020
(73) Patentinhaber: Robert Bosch GmbH, 70442 Stuttgart (DE)
(72) Erfinder: HEUSSNER, Nico, 71636 Ludwigsburg (DE); REPPICH, Raimund, 71636 Ludwigsburg (DE); BLASE, Benjamin, 74321 Bietigheim-Bissingen (DE)
(86) Internationale Anmeldenummer: PCT/EP2019/051004
(87) Internationale Veröffentlichungsnummer: WO 2019/149523

(56) Entgegenhaltungen:
- WO-A1-03/101325
- US-A1- 2012 296 318
- US-B2- 9 579 153

## Beschreibung

### Stand der Technik

Die vorliegende Erfindung betrifft ein Verfahren zum Aussenden von Laserlicht. Insbesondere wird durch das Verfahren sichergestellt, dass eine Augensicherheit beim Aussenden des Laserlichts erreicht wird. Des Weiteren betrifft die Erfindung ein Computerprogrammprodukt sowie ein Lasersystem zum Aussenden von Laserlicht.

Aus dem Stand der Technik sind optisch aktive Sensoren und Aktoren bekannt, die eine aktive Lichtquelle, wie insbesondere einen Laser, einsetzen. Solche Sensoren und Aktoren müssen derart ausgelegt werden, dass die maximale imitierte Lichtleistung auch in einer ungünstigen räumlichen und zeitlichen Konstellation ein menschliches Augenlicht beschädigen kann.

Hierfür existieren internationale Normen zur Augensicherheit, anhand derer abhängig von den Parametern der Lichtemission Lichtleistungsgrenzwerte zur garantierten Einhaltung der Augensicherheit in allen denkbaren Betriebsszenarien abgeleitet werden können. Die Annahme der ungünstigsten räumlichen und zeitlichen Konstellation eines solchen Systems zu jedem Zeitpunkt führt dazu, dass die Parameter der Lichtemissionen bei einem normalen Betrieb unterhalb der Schädigungsgrenze betrieben werden. Es findet insbesondere keine Betrachtung des tatsächlichen Betriebsverhaltens statt, so dass die Aussendung von Laserlicht einer Steuerung folgt, die fest vorgegebene Grenzwerte beinhaltet.

Aus der DE 10 2007 018 366 ist ein System zum betreiben einer Laservorrichtung bekannt. Dabei ist vorgesehen, dass eine Steuerung vorhanden ist, die ein Laserpulsfolgensignal liefert, dass einen festgelegten Arbeitszyklus und einen festgelegten Leistungspegel aufweist, das insbesondere

Anforderungen an eine Augensicherheitsgrenze erfüllt. Aus der US9579153 ist ein System zur Augenchirurgie bekannt.

### Offenbarung der Erfindung

Die Erfindung ermöglicht durch eine aktive Regelung von Betriebsparametern, dass die Augensicherheit für die jeweils aktuelle Gefährdungssituation sichergestellt wird. Somit kann bei regulärem Betrieb eine höhere Leistung abgegeben werden, als dies im Stand der Technik möglich ist. Dies ist sowohl für optische Aktoren, wie beispielsweise Laserprojektoren, als auch für optisch aktive Sensoren, wie beispielsweise LiDAR-Systemen, von Vorteil. Konkret werden durch die aktive Regelung gewisse Worst-Case-Szenarien entweder aktiv abgefangen, so dass die zulässige Leistung sich erhöht oder eine adaptive Anpassung der Ausgangsleistung durchgeführt. Letzteres führt insbesondere zu einer Absenkung der Emissionsleistung, falls das Betriebsverhaltung in Richtung eines Worst-Case-Szenarios läuft.

Das erfindungsgemäße Verfahren zum Aussenden von Laserlicht in Form von Laserpulsen umfasst die folgenden Schritte:
Zunächst erfolgt ein Planen eines Laserpulses anhand von Pulsparametern. Die Pulsparameter stellen Eigenschaften des Laserpulses dar, anhand derer eine Aussendeeinheit angesteuert werden muss, um den Laserpuls auszusenden. Anschließend erfolgt ein Überprüfen, ob Laserpulse, die innerhalb eines vordefinierten vorangegangenen Zeitintervalls ausgesandt wurden, zusammen mit dem geplanten Puls ein vordefiniertes Energiekriterium erfüllen. Durch das Energiekriterium ist insbesondere eine Augensicherheit gewährleistet. Dies bedeutet, dass das vordefinierte Energiekriterium solche Energiegrenzwerte beinhaltet, die für eine Augensicherheit notwendig sind. Somit wird erfindungsgemäß für jeden Puls überprüft, ob dieser das Energiekriterium für die Augensicherheit einhält. Dabei wird auf die tatsächlich ausgesandten vorherigen Pulse zurückgegriffen, so dass ein sehr genaues Bild über die bisher ausgesandte Energie in Form von Laserlicht ermittelt werden kann. Zuletzt erfolgt das Aussenden des geplanten Laserpulses mittels der Aussendeeinheit, wenn das Energiekriterium erfüllt ist. Ist das Energiekriterium hingegen nicht erfüllt, so wird der geplante Laserpuls nicht ausgesandt. Alternativ erfolgt ein Verringern einer Leistung des Laserpulses, wenn das Energiekriterium nicht erfüllt ist. Dies bedeutet, dass die Pulsparameter im Falle des Nichterfüllens des Energiekriteriums verändert werden können und anstelle des geplanten Laserpulses wird ein Laserpuls mit einer geringeren Leistung ausgesandt. Dies bedeutet, dass für jeden Laserpuls überprüft wird, ob das Aussenden des geplanten Laserpulses zu einer Verletzung des Energiekriteriums führen würde, was bedeuten würde, dass Augensicherheit nicht mehr gewährleistet sein kann. Es erfolgt somit eine stetige Überwachung der laseremittierenden Systems, insbesondere der Aussendeeinheit. Während im Gegensatz dazu gängige Systeme für die Augensicherheit einmalige Berechnungen unter Annahme von Worst-Case-Szenarien anstellen, um die Parameter für Emissionsgrenzen und damit auch die Parameter für das laseremittierende System in sämtlichen Betriebszuständen festlegen, wird im Rahmen der Erfindung eine tatsächlich emittierte Strahlung, insbesondere zusammen mit einem Systemzustand der Aussendeeinheit, registriert, um daraus Berechnungen anzustellen, welche Parameter das laseremittierende System in einem augensicheren Zustand halten. Diese Berechnungen werden vorteilhafterweise in Echtzeit auf Basis der Parameter durchgeführt. Dies bedeutet, dass das erfindungsgemäße Verfahren in Echtzeit für jeden geplanten Laserpuls durchgeführt wird. Somit findet eine Regelung statt.

Die Unteransprüche haben bevorzugte Weiterbildungen der Erfindung zum Inhalt.

Bevorzugt ist vorgesehen, dass das Energiekriterium eine Maximalenergie des Laserlichts darstellt, die nicht zu bleibenden Schäden bei einem menschlichen Auge führt. Insbesondere sind aus dem Stand der Technik Augensicherheitsnormen bekannt. Diese Augensicherheitsnormen geben an, welche Lichtleistung ein menschliches Auge treffen darf, ohne dass dies zu bleibenden Schäden führt. Diese maximale Lichtleistung lässt sich zu einem Energiekriterium formen, das auf eine spezifische Aussendeeinheit angepasst ist. Besonders vorteilhaft umfasst das Energiekriterium drei verschiedene Regeln: Eine erste Regel ist ein Einzelpulskriterium, welches durch die Überwachung der Pulsparameter eines einzelnen Laserpulses sichergestellt werden kann. Die zu betrachtenden Pulsparameter sind insbesondere eine Pulsdauer und eine Pulsenergie. Eine zweite Regel ist ein Mittelwertkriterium, welches die Abfolge von Laserpulsen, was im folgenden auch als Pulsmuster beschrieben wird, limitiert. Hierbei wird ein Zeitfenster einer beliebigen Länge über das Pulsmuster gelegt und die darin emittierte Energie aufintegriert. Die freigesetzte Energie darf eine Grenze nicht überschreiten, welche von der Länge des Zeitfensters abgeleitet werden kann. Dieser Vorgang muss für jede Zeitdauer zwischen maximal 1 × 10⁻⁷ s und 30.000 s und jeden Startpunkt während des Pulsmusters gelten. Eine dritte Regel ist ein Reduktionskriterium, bei welchem analysiert wird, wie viele theoretische Pulse einer gewissen Länge in das maximal zu betrachtende Zeitfenster aus der zweiten Regel passen. Aus der Länge dieser Quasipulse und ihrer Anzahl wird ein Korrekturfaktor errechnet, welcher zu der Energiegrenze der zweiten Regel multipliziert wird.

In einer weiteren bevorzugten Ausführungsform ist vorgesehen, dass sämtliche Laserpulse diskrete Laserpulse sind. Dies vereinfacht die Ermittlung und Überprüfung des Energiekriteriums. So wurde insbesondere zuvor beschrieben, dass drei verschiedene Regeln eingehalten werden sollen. Die Einhaltung dieser Regeln kann beispielsweise nach einer Brute-Force-Methode berechnet werden, bei welcher jeder einzelne Puls gespeichert wird und unter hohem Ressourcenaufwand die Zeitfenster berechnet werden, die zum Überprüfen des Einhaltens der Energiegrenzen notwendig sind. Zudem muss bei scannenden Systemen zusätzlich der Ausfallswinkel der Laserpulse berücksichtigt werden, um so nicht nur über die gesamte Zeit, sondern auch über den gesamten Winkelbereich der Emission iteriert werden. Um dies zu vereinfachen, ist vorgesehen, dass lediglich diskrete Laserpulse vorliegen, wodurch insbesondere keine zeitlich konstante abgestrahlte Welle (continuous wave, cw) oder ein modulierter Strahl ausgesandt wird. Zwischen zwei diskreten Laserpulsen liegt besonders vorteilhaft ein vordefinierter minimaler Abstand vor. Dies vereinfacht insbesondere die Überprüfung des Energiekriteriums, da insbesondere die maximale Anzahl von Laserpulsen innerhalb eines vordefinierten Zeitfensters aufgrund des vordefinierten minimalen Abstands der Laserpulse einen oberen Grenzwert aufweist. Des Weiteren lässt sich die emittierte Lichtleistung einfach und aufwandsarm ermitteln, indem die Einzelenergien der Laserpulse aufintegriert wird. Somit ist ein ressourcen-schonendes und schnelles Überprüfen des Einhaltens oder Nichteinhaltens des Energiekriteriums ermöglicht.

In einer bevorzugten Ausführungsform ist vorgesehen, dass das Energiekriterium einen Maximalwert einer erlaubten Pulsdauer und/oder einer Pulsenergie des geplanten Laserpulses aufweist. Alternativ oder zusätzlich ist vorgesehen, dass das Energiekriterium einen Minimalwert eines Abstands zwischen zwei Laserpulsen aufweist. Dies bedeutet, dass das Energiekriterium ein Einzelpulskriterium wie zuvor als erste Regel beschrieben umfasst. Insbesondere wird überprüft, ob der einzelne geplante Laserpuls bei Aussendung bereits selbst das Energiekriterium verletzen würde. Eine Betrachtung der vorausgegangenen ausgesandten Laserpulse ist hierzu nicht notwendig.

Weiterhin ist vorteilhafterweise vorgesehen, dass ein vordefiniertes Pulsmuster wiederholend ausgesandt wird. Dies bedeutet, dass ein Frame vorliegt, das sich in den ausgesandten Laserpulsen stets wiederspiegelt. Insbesondere kann das sich wiederholende Pulsmuster ein Bild eines Laserprojektors oder ein Frame in einem LiDAR-Sensor sein. Somit wird eine Periodizität des ausgesandten Laserlichts erreicht. Dies führt wiederum zu einer Vereinfachung der Überprüfung des Energiekriteriums, da insbesondere die zweite Regel wie zuvor beschrieben auf das Überprüfen der Lichtleistung der sich wiederholenden Pulsmuster beschränkt werden kann. Somit lässt sich ressourcen-schonend und schnell überprüfen, ob das Energiekriterium erfüllt ist.

Besonders vorteilhaft umfasst das Energiekriterium einen Maximalwert einer Emissionsenergie des gesamten Pulsmusters. Somit wird insbesondere für jedes Pulsmuster überprüft, ob ein Maximalwert einer Lichtleistung eingehalten wird. Ist der Maximalwert erreicht, so werden insbesondere keine weiteren Pulse des Pulsmusters ausgesandt. Dies bedeutet, dass geplante Laserpulse des Pulsmusters nicht ausgesandt werden. Alternativ kann eine Annäherung an den Maximalwert erkannt werden und das Aussenden weiterer geplanter Laserpulse des Pulsmusters nur unter reduzierter Emissionsenergie erlaubt werden. Dies bedeutet, dass die Energie der einzelnen geplanten Laserpulse reduziert wird, um sämtliche Laserpulse des Pulsmusters auszusenden, den Maximalwert der Emissionsenergie des Pulsmusters dennoch zu erfüllen.

Bevorzugt wird das Laserlicht unter einem sich verändernden Raumwinkel scannend ausgesandt. Der Raumwinkel wird in ein Raster aus diskreten Pixeln diskretisiert. Für jeden Pixel wird eine Lichtleistung jedes den Pixel treffenden Laserpulses aufintegriert, um das Erfüllen des Energiekriteriums für jeden Pixel zu überprüfen. Insbesondere wird eine solche Berechnung auch für den geplanten Laserpuls durchgeführt. Anhand der Diskretisierung mittels Pixel lässt sich auch ein maximaler Augendurchmesser eines menschlichen Auges diskretisieren. Somit kann diejenige Fläche, über die das Laserlicht in das menschliche Auge einfallen kann, durch eine Anzahl von Pixeln diskretisiert werden. Daraus lässt sich diejenige Anzahl an Pixeln ermitteln, die bei einem scannenden System maximal von den einzelnen Laserpulsen getroffen werden. Daraus lässt sich wiederum einfach und aufwandsarm ableiten, wie viel Lichtenergie auf einen einzelnen Pixel treffen darf, um Augensicherheit zu gewährleisten. Somit ist das Energiekriterium anhand der Diskretisierung einfach und aufwandsarm ermittelbar. Insbesondere ist eine Größe der Pixel deutlich kleiner als eine Augenfläche oder des Querschnitts des ausgesandten Laserlichts. Ein mittlerer Diskretisierungsfehler sinkt mit der Pixeldichte, wobei hingegen auch Rechenaufwand sowie Ressourcenaufwand ansteigen. Die entsandte Lichtleistung eines Laserpulses wird somit auf jeden betroffenen Pixel aufgeteilt. Es lässt sich somit für jeden Pixel einfach und aufwandsarm ermitteln, welcher Lichtenergie dieser bei einem einzelnen Laserpuls oder bei einem Pulsmuster ausgesetzt ist.

Besonders vorteilhaft wird ein Zeitbereich bestimmt, während dem das Laserlicht eine vordefinierte Augenöffnungsfläche überstreicht. Die Augenöffnungsfläche stellt diejenige Fläche dar, durch die das Laserlicht in ein menschliches Auge gelangen kann. Die Augenöffnungsfläche wird definiert durch den Durchmesser der Pupille des Auges. Während dieses Zeitbereichs ist eine Pulsgruppe von mehreren Laserpulsen aussendbar. Das Energiekriterium umfasst vorteilhafterweise einen Maximalwert für die Anzahl von Laserpulsen der Pulsgruppe und/oder für einen Abstand zweier aufeinanderfolgender Pulsgruppen.

Die Pulsparameter umfassen vorteilhafterweise einen Pulszeitpunkt und/oder eine Pulsenergie und/oder eine Pulslänge und/oder eine Pulsleistung und/oder einen Pulsaustrittswinkel. Anhand dieser Parameter wird der geplante Laserpuls geplant und anhand dieser Parameter wird der geplante Laserpuls geplant und anhand dieser Parameter wird überprüft, ob der geplante Laserpuls das Energiekriterium erfüllt. Besonders vorteilhaft können zusätzlich Systemparameter der Aussendeeinheit ausgelesen werden. Solche Systemparameter umfassend insbesondere eine Ausrichtung, eine Eigenbewegung, insbesondere Vibrationen und Querbeschleunigung, Daten einer Abstandssensorik oder aktuelle Informationen über die Optik und Strahlformung. Diese Systemparameter geben Aufschluss darüber ob sie der Systemzustand verändert hat und ob und wie die Berechnung der Augensicherheit angepasst werden muss.

Die Erfindung betrifft des Weiteren ein Computerprogrammprodukt. Das Computerprogrammprodukt umfasst auf einem maschinenlesbaren Speichermedium gespeicherten Programmcode zur Durchführung des zuvor beschriebenen Verfahrens, wenn das Computerprogrammprodukt auf einer Rechenvorrichtung abläuft. Die Rechenvorrichtung ist vorteilhafterweise ein Steuergerät eines laseremittierenden Systems. Beispielsweise kann die Rechenvorrichtung die Steuervorrichtung eines Laserprojektors oder eines LiDAR-Sensors sein.

Zuletzt betrifft die Erfindung ein Lasersystem zum Aussenden von Laserlicht. Das Lasersystem umfasst eine Aussendeeinheit zum Aussenden von Laserpulsen anhand von Pulsparametern. Die Pulsparameter entsprechen insbesondere den zuvor beschriebenen Pulsparametern. Des Weiteren umfasst das Lasersystem ein Steuergerät zum Ansteuern der Aussendeeinheit. Das Steuergerät ist ausgebildet, ein Verfahren wie zuvor beschrieben durchzuführen. Insbesondere ist das Steuergerät eine Rechenvorrichtung, wie zuvor definiert.

### Zeichnungen

Nachfolgend werden Ausführungsbeispiele der Erfindung unter Bezugnahme auf die begleitende Zeichnung im Detail beschrieben. In der Zeichnung ist:
- Figur 1: eine schematische Ansicht eines Lasersystems gemäß einem Ausführungsbeispiel der Erfindung,
- Figur 2: eine schematische Ansicht einer Diskretisierung von Raumwinkeln des mittels des Lasersystems aus Figur 1 ausgesandten Laserlichts,
- Figur 3: eine erste schematische Ansicht der Herleitung des Energiekriteriums,
- Figur 4: eine zweite schematische Ansicht der Herleitung des Energiekriteriums,
- Figur 5: eine dritte schematische Ansicht der Herleitung des Energiekriteriums, und
- Figur 6: eine vierte schematische Ansicht der Herleitung des Energiekriteriums.

### Ausführungsformen der Erfindung

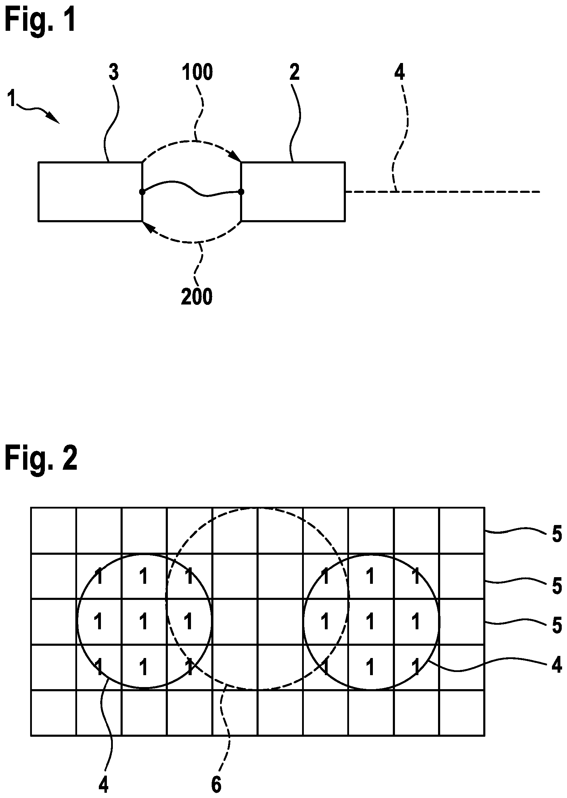

Figur 1 zeigt schematisch ein Lasersystem 1 zum Aussenden von Laserlicht 4. Das Lasersystem 1 umfasst eine Aussendeeinheit 2 zum Aussenden von Laserpulsen anhand von Pulsparametern. Durch die Aussendeeinheit 2 werden nur diskrete Laserpulse mit einem vordefinierten minimalen Abstand ausgesandt.

Des Weiteren weist das Lasersystem 1 ein Steuergerät 3 auf. Das Steuergerät 3 dient zum Ansteuern der Aussendeeinheit 2. Dabei stellt das Steuergerät 3 durch ein Verfahren gemäß einem Ausführungsbeispiel der Erfindung sicher, dass das ausgesandte Laserlicht 4 augensicher ist, d.h., dass das ausgesandte Laserlicht 4 ein menschliches Auge nicht bleibend schädigen kann. Gleichzeitig ist eine Ausgangsleistung des Laserlichts 4 maximiert.

Das Steuergerät 3 plant einen von der Aussendeeinheit 2 auszusenden Laserpuls und steuert die Aussendeeinheit 2 mit entsprechendem Pulsparametern 100 an, damit diese den Laserpuls als Laserlicht 4 aussendet. Dabei überprüft das Steuergerät 3 zusätzlich, ob der geplante Laserpuls ein vordefiniertes Energiekriterium verletzen würde. Ist dies der Fall, so werden entweder die Pulsparameter 100 modifiziert, um eine Ausgangsleistung des geplanten Laserpulses zu verringern, oder es wird gänzlich auf das Aussenden des geplanten Laserpulses verzichtet.

Um das Einhalten oder Nichteinhalten des Energiekriteriums überprüfen zu können, werden insbesondere Messparameter 200 von der Aussendeeinheit 2 ausgelesen. Diese Messparameter 200 umfassen insbesondere die Pulsparameter 100 der bereits ausgesandten Laserpulse und umfassen zusätzlich Systemparameter. Bei den Pulsparametern handelt es sich um einen Pulszeitpunkt und/oder eine Pulsenergie und/oder eine Pulslänge und/oder eine Pulsleistung und/oder einen Austrittswinkel. Bei den Systemparametern handelt es sich insbesondere um Parameter wie Ausrichtung und/oder Eigenbewegung und/oder Daten einer Abstandssensorik und/oder aktuelle Informationen über eine Optik und Strahlformung. Bei der Eigenbewegung werden insbesondere Vibration und/oder Querbeschleunigung bestimmt. Diese Systemparameter lassen Rückschlüsse zu, ob der Systemzustand sich verändert hat. Außerdem kann anhand der Systemparameter ermittelt werden, ob und wie die Berechnung der Augensicherheit angepasst werden muss.

Grundsätzlich hat das Energiekriterium festzulegen, welche maximale Energie ein zu einem beliebigen Zeitpunkt ausgesandter Laserpuls aufweisen darf, um Augensicherheit zu gewährleisten. Die Überprüfung der Augensicherheit erfolgt vorteilhafterweise anhand von drei verschiedenen Regeln:
Eine erste Regel stellt ein Einzelpulskriterium dar. Bei dem Einzelpulskriterium wird überprüft, ob der einzelne geplante Laserpuls eine zu hohe Energie aufweist, dass bereits der einzelne Laserpuls selbst zu einer Verletzung der Augensicherheit führen würde. Somit lässt sich die erste Regel insbesondere durch Überwachung der Pulsparameter der geplanten Laserpulse sicherstellen. Insbesondere werden eine Pulsdauer und eine Pulsenergie überprüft.

Eine zweite Regel ist ein Mittelwertkriterium, welches die Abfolge von Pulsen eines Pulsmusters limitiert. Dabei ist vorgesehen, dass eine Abfolge von Laserpulsen als Pulsmuster angesehen werden. Hierbei wird ein Zeitfenster einer beliebigen Länge über das Pulsmuster gelegt und die darin emittierte Energie aufintegriert. Die freigesetzte Energie darf eine Grenze nicht überschreiten, welche sich aus der Länge des Zeitfensters ableitet. Dieser Vorgang muss für beliebige Zeitdauern und für beliebige Startpunkte gelten.

Eine dritte Regel ist ein Reduktionskriterium, bei dem analysiert wird, wie viele theoretische Impulse einer gewissen Länge in das maximal zu betrachtende Zeitfenster aus der vorherigen zweiten Regel passen. Aus der Länge dieser Quasipulse und ihrer Anzahl lässt sich ein Korrekturfaktor errechnen, welcher zu der Energiegrenze aus der zweiten Regel multipliziert wird.

Das Einhalten dieser Regeln kann durch eine Brute-Force-Methode überprüft werden, bei der jeder einzelne Puls gespeichert wird und unter relativ hohem Ressourcenaufwand einzelne Zeitfenster berechnet werden, anhand derer die Energiegrenzen überprüft werden können. Dies ist allerdings nicht praktikabel. Daher ist vorgesehen, dass die Aussendeeinheit 2 Laserlicht 4 nur unter den folgenden Voraussetzungen aussendet:
Es werden nur diskrete Laserpulse ausgesandt, so dass keine kontinuierliche Welle oder ein modulierter Strahl vorhanden sind. Zwischen zwei dieser diskreten Laserpulse ist ein vordefinierter minimaler Pulsabstand vorhanden. Das Aussenden des Laserlichts 4 folgt insbesondere unter wiederholenden Pulsmustern, was bedeutet, dass das Laserlicht 4 eine Periodizität aufweist. Insbesondere setzt sich das Laserlicht somit aus einer Folge sich wiederholender Pulsmuster zusammen. Das Pulsmuster kann beispielsweise das Bild eines Laserprojektors oder das Frame eines LiDAR-Sensors sein.

Anhand dieser Vereinfachungen lässt sich das Energiekriterium, insbesondere unter Berücksichtigung der drei vorgenannten Regeln einfach und aufwandsarm überprüfen. Hierzu ist ein erster zeitlicher Überwachungsbereich vorgesehen, der sich aus der Periodizität des Laserlichts 4 ableitet. Anhand jedes Pulsmusters lässt sich eine maximale Emissionsenergie ermitteln, die für die Augensicherheit erlaubt ist. So ist insbesondere ermöglicht, für jedes Pulsmuster einen eigenen Integrator zu starten, um die gesamte in dem Pulsmuster emittierte Energie zu ermitteln. Diese Energie darf einen Grenzwert nicht überschreiten, wobei sich der Grenzwert aus bekannten Augensicherheitsnormen ableiten lässt. Würde dieser Grenzwert durch einen geplanten Laserpuls überschritten, so wird entweder eine Ausgangsleistung des Laserpulses reduziert oder es wird gänzlich auf das Aussenden des Laserpulses verzichtet. Ebenso ist vorteilhafterweise vorgesehen, dass bei einer Annäherung an den Grenzwert das Steuergerät 3 die Ausgangsleistung der geplanten Laserpulse reduziert, auch wenn mit dem aktuell geplanten Laserpuls der Maximalwert für die Energie des Pulsmusters nicht überschritten würde.

Ein zweiter zeitlicher Überwachungsbereich leitet sich daraus ab, dass in einem scannenden System das Laserlicht 4 ein in den Projektionsraum platziertes menschliches Auge überstreicht. Insbesondere ist hierzu die Aussendeeinheit 2 eingerichtet, das Laserlicht unter einem variablen Winkel auszusenden. Dies führt zu einer Pulsgruppe, die in einem Augenüberstrich in ein Auge fallen könnte. Es lässt sich somit anhand einer Bewegungsgeschwindigkeit, unter der sich der Winkel des ausgesandten Laserlichts 4 verändert, eine maximale Anzahl von Laserpulsen ermitteln, die in das Auge fallen können. Insbesondere wird der zweite Überwachungsbereich an der maximalen zeitlichen Dauer des Augenüberstrichs orientiert. Somit lässt sich eine Energiegrenze ermitteln, den besagte Pulsgruppe maximal aufweisen darf. Das Überwachen des zweiten Überwachungsbereichs erfolgt analog zu dem ersten Überwachungsbereich durch einen Integrator, der die Energie der Laserpulse der Pulsgruppe aufintegriert. Die Energiegrenze des zweiten zeitlichen Überwachungsbereichs lässt sich vorteilhafterweise aus bekannten Augensicherheitsnormen ableiten.

Ein dritter zeitlicher Überwachungsbereich orientiert sich an einem einzelnen geplanten Laserpuls und dessen Abstand zum vorherigen ausgesandten Laserpuls. So muss hier für jeden einzelnen Laserpuls überprüft werden, ob die geplante Pulsenergie eine Augensicherheit gewährleistet oder ob Augensicherheit durch besagte Pulsenergie nicht mehr gewährleistet ist. Gleiches gilt für einen Abstand zu einem vorausgegangenen ausgesandten Laserpuls, wobei sich auch für diese Fälle aus bekannten Augensicherheitsnormen Grenzwerte ableiten lassen, die von den einzelnen Laserpulsen einzuhalten sind.

In Figur 2 ist schematisch eine Diskretisierung gemäß dem Ausführungsbeispiel der Erfindung gezeigt. Dies ist notwendig, da die Augensicherheitsberechnungen immer für den Lichteinfall in einen Augendurchmesser durchgeführt werden müssen. Es ist deshalb ein System zu entwickeln, dass diese Parameter modular für jeden möglichen im Winkelraum platzierten Augendurchmesser berechnet. Daher ist ein Raster aus diskreten Pixeln 5 vorgesehen, wobei die Pixel 5 die Größe eines Bruchteils einer Augenöffnungsfläche 6 aufweisen. Die Augenöffnungsfläche 6 wird bestimmt durch einen maximalen Durchmesser einer Pupille eines zu schützenden Auges. Die Lichtleistung des Laserlichts 4 wird gemäß den drei zuvor beschriebenen zeitlichen Überwachungsbereichen anteilsmäßig in den entsprechenden Pixeln 5, die von dem Laserlicht 4 betroffen sind, akkumuliert. Die oben beschriebene Berechnung der zeitlich akkumulierten Lichtleistung im Sinne der Augensicherheit kann für jedes mögliche räumliche Integralfenster der Größe eines Augendurchmessers erfolgen. Der mittlere Diskretisierungsfehler eines solchen Systems sinkt mit der Pixeldichte des Rasters, womit hingegen auch der Rechenaufwand sowie Ressourcenaufwand ansteigt.

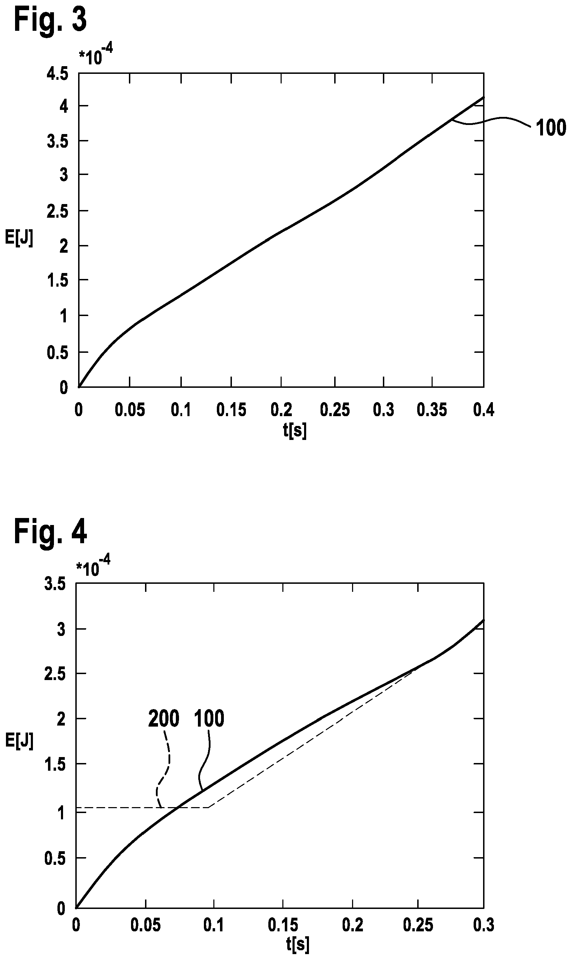

Die Figuren 3 bis 6 zeigen schematisch eine exemplarische Herleitung des Energiekriteriums für Laserlicht 4 mit einer Wellenlänge von 905 Nanometern. Bei dieser Wellenlänge wächst das Energielimit gemäß bekannter Augensicherheitsnormen über die Dauer des betrachteten Zeitfensters zunächst mit einer Wurzelfunktion und steigt daher am Anfang stark an, gegen Ende hin aber immer weniger. Zusätzlich schlägt sich ab einer gewissen Zeit der Korrekturfaktor aus der zuvor beschriebenen dritten Regel nieder, so dass die Kurve kein einfach zu berechnendes Profil aufweist. Der Verlauf des Energielimits 100 ist in Figur 3 schematisch dargestellt.

Zunächst wird ein erster Vereinfachungsbereich ermittelt, der sich darauf bezieht, dass Pulsmuster eine Periodizität aufweist. Dieser wiederholte Zeitabschnitt hat eine gewisse Dauer und eine gleichbleibende emittierte Energie und soll beliebig oft wiederholt werden können, ohne die Augensicherheit zu verletzen. Daher wird das höchste bekannte Limit linear heruntergebrochen, auf die Länge einer eines Pulsmusters. Dieses höchste bekannte Limit ist die Definition der Grenze über das größtmögliche Zeitfenster. Durch das lineare Herunterbrechen wird eine Energiegrenze 200 festgesetzt, welche pro Frame pro Winkelbereich, den ein Auge einnimmt, emittiert werden darf. Der Winkelbereich entspricht dem Winkelbereich desjenigen Aussendewinkels, unter dem das Laserlicht 4 von der Aussendeeinheit 2 ausgesandt wird.

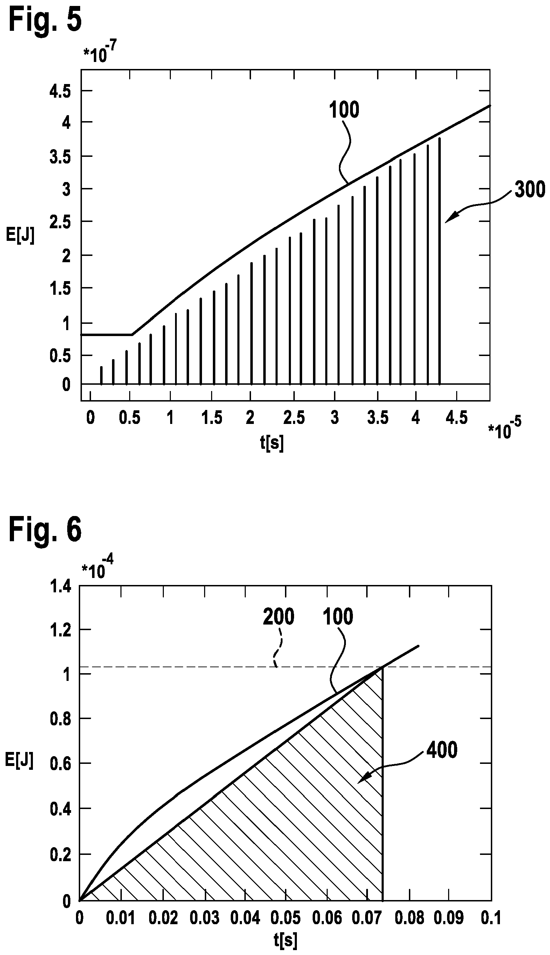

Ein zweiter Bereich der Vereinfachung bezieht sich auf die Annahme, dass es das Ziel des Lasersystems 1 ist, in kurzer Zeit viele Laserpulse konsekutiv hintereinander in einen ähnlichen Winkelbereich zu emittieren. Dafür wird eine Pulsgruppe definiert, welche die maximale Anzahl an Laserpulsen festlegt, die nacheinander mit dem vordefinierten minimalen Pulsabstand emittiert werden können. Die maximal emittierbare Pulsanzahl 300 ist in Figur 5 gezeigt.

Ein dritter Bereich ergibt sich aus der Zusammenführung des ersten Bereichs wie in Figur 4 gezeigt und des zweiten Bereichs wie in Figur 5 gezeigt. Auf diese Weise ist ein minimaler Bereich der Augensicherheitsnorm durch die Pulsgruppe abgedeckt und eine absolute Energiegrenze pro Pulsmuster festgesetzt. Daraus lässt sich ableiten, wie lange nach jeder Pulsgruppe mindestens gewartet werden muss, um innerhalb eines Pulsmusters das Energielimit 100 nicht zu überschreiten.

Figur 6 zeigt hierzu schematisch die emittierten Pulsgruppen 400 bis zum Erreichen der Energiegrenze 200 des Pulsmusters.

Die zuvor beschriebenen Vereinfachungen ermöglichen daher, dass Pulsgruppen mit einem bestimmten zeitlichen Abstand auszusenden sind, während gleichzeitig eine Energiegrenze pro Pulsmuster überwacht wird. Die Paramater dieser Vereinfachung lauten daher wie folgt:
- maximal zulässige Einzelpulsenergie
- minimal zulässiger zeitlicher Einzelpulsabstand
- maximale zulässige Anzahl von Einzelpulsen innerhalb einer Pulsgruppe
- minimal zulässiger zeitlicher Abstand zwischen zwei Pulsgruppen
- maximal zulässige Energiegrenze pro Frame (maximal zulässige Pulsanzahl pro Frame)

Aus einer vorgegebenen Pulslänge und Pulsleistung kann die Pulsenergie eines geplanten Laserpulses sowie eines ausgesandten Laserpulses hergeleitet werden. Daraus kann die maximal zulässige Energiegrenze pro Pulsmuster wie zuvor beschrieben berechnet werden. Der minimal zulässige Einzelpulsabstand kann aus der Zeit gewonnen werden, wenn das Lasersystem 1 ein scannendes System ist, die das System in einen Augendurchmesser ohne Unterbrechung Pulse emittiert. So kann eine maximale Energie berechnet werden, die in dieser Zeit emittiert werden darf, woraus die Anzahl der Laserpulse und daraus wiederum der minimal zulässige Einzelpulsabstand resultiert. Die Anzahl der Laserpulse entspricht gleichzeitig der maximal zulässigen Anzahl von Einzelpulsen innerhalb einer Pulsgruppe.

Der minimal zulässige zeitliche Abstand zwischen zwei Pulsgruppen wird berechnet aus dem Schnittpunkt des Energielimits 100 mit dem maximalen Energielimit 200 pro Pulsmuster. Alle Laserpulse, die die Energie über das Energielimit 200 heben würden, werden bereits durch diesen Mechanismus verhindert. Deshalb wird bis zu dem Zeitpunkt, an dem das Energielimit 200 erreicht wird, eine Überwachung sichergestellt. Aus dem Zeitpunkt, an dem das Energielimit 200 erreicht wird, der maximal zulässigen Energiegrenze und den Pulsgruppenparametern kann der minimal zulässige zeitliche Abstand zwischen zwei Pulsgruppen ermittelt werden.

Besonders vorteilhaft kann auf alle verwendeten Parameter ein Sicherheitsabstand addiert werden. Dies führt dazu, dass in einem Fehlerfall nicht sofort die Grenzen von bekannten Augensicherheitsnormen erreicht werden. Somit ist ein Puffer vorhanden, der die Augensicherheit des Lasersystems 1 erhöht.

Im Folgenden wir mittels Pseudocode ein möglicher Algorithmus dargestellt, um das erfindungsgemäße Verfahren auf dem Steuergerät 3 zu implementieren:

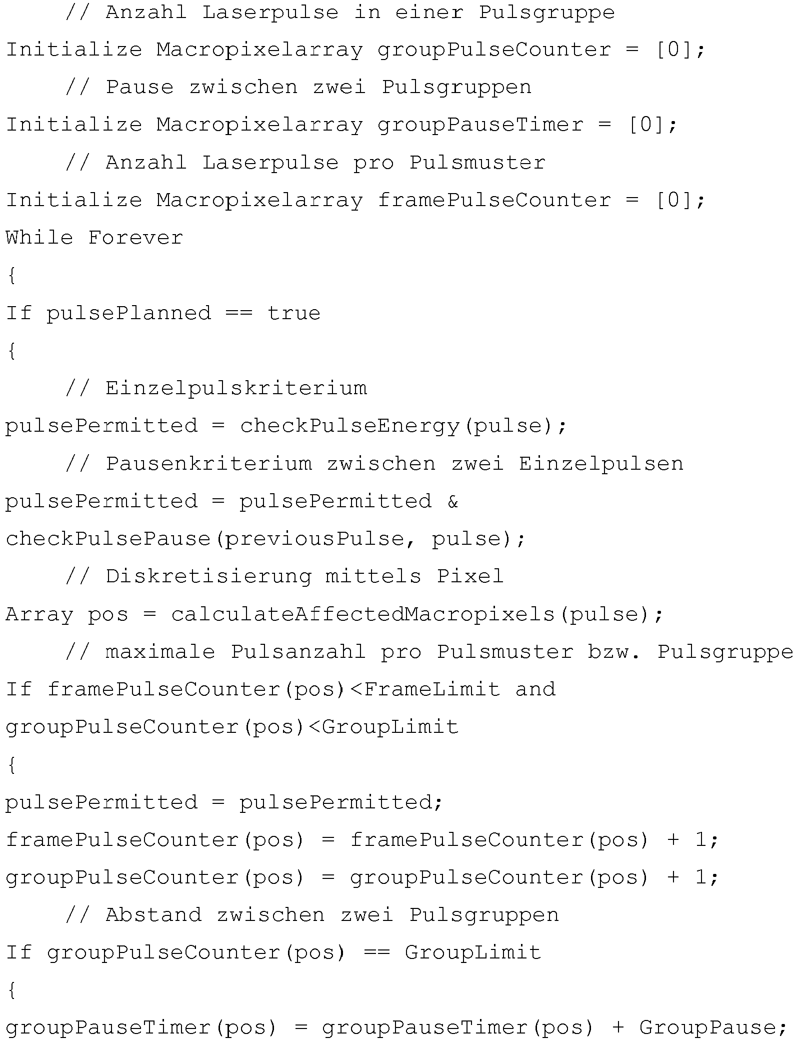

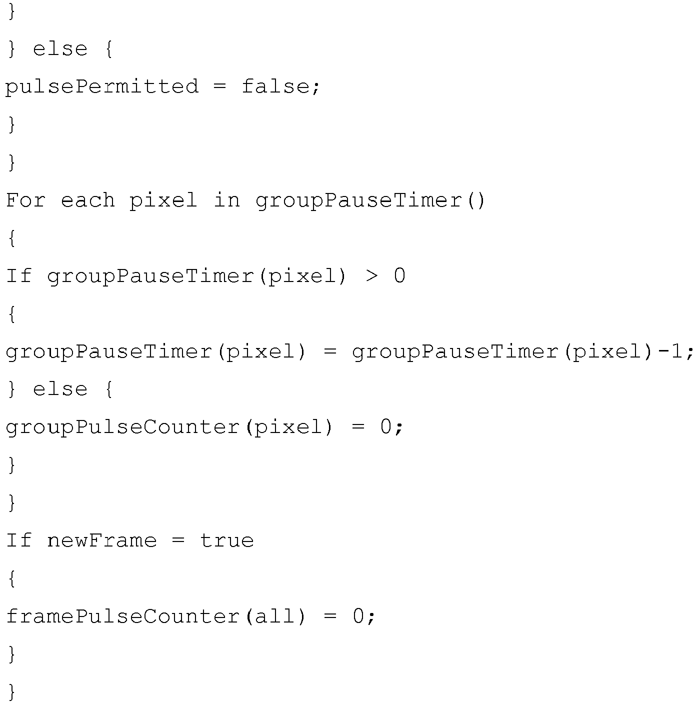

## Patentansprüche

1. Verfahren zum Betrieb eines LiDAR-Systems durch Aussenden von Laserlicht (4) in Form von Laserpulsen umfassend die Schritte
• Planen eines Laserpulses anhand von Pulsparametern (100),
• Berechnen ob Laserpulse, die innerhalb eines vordefinierten vorangegangenen Zeitintervalls ausgesandt wurden, zusammen mit dem geplanten Puls ein vordefiniertes Energiekriterium erfüllen, wobei die Berechnung in Echtzeit durchgeführt wird und
• Aussenden des geplanten Laserpulses mittels einer Aussendeeinheit (2), wenn das Energiekriterium erfüllt ist, und nicht Aussenden des geplanten Laserpulses oder Verringerung einer Leistung des Laserpulses, wenn das Energiekriterium nicht erfüllt ist, wobei ein vordefiniertes Pulsmuster wiederholend ausgesandt wird und wobei das Energiekriterium einen Maximalwert einer Emissionsenergie des gesamten Pulsmusters umfasst
oder
wobei das Energiekriterium einen Maximalwert einer erlaubten Pulsdauer und/oder Pulsenergie des geplanten Laserpulses und/oder einen Minimalwert eines Abstands zwischen zwei Laserpulsen umfasst.

2. Verfahren nach Anspruch 1, **dadurch gekennzeichnet, dass** das Energiekriterium eine Maximalenergie des Laserlichts darstellt, die nicht zu bleibenden Schäden bei einem menschlichen Auge führt.

3. Verfahren nach einem der vorhergehenden Ansprüche, **dadurch gekennzeichnet, dass** sämtliche Laserpulse diskrete Laserpulse, insbesondere mit einem vordefinierten minimalen Abstand, sind.

4. Verfahren nach einem der vorhergehenden Ansprüche, **dadurch gekennzeichnet, dass** das Laserlicht unter einem sich verändernden Raumwinkel scannend ausgesandt wird, wobei der Raumwinkel in ein Raster aus diskreten Pixeln (5) diskretisiert wird, wobei für jeden Pixel (5) eine Lichtleistung jedes den Pixel (5) treffenden Laserpulses aufintegriert wird, und wobei das Erfüllen des Energiekriteriums für jeden Pixel (5) überprüft wird.

5. Verfahren nach Anspruch 4 **dadurch gekennzeichnet, dass** ein Zeitbereich bestimmt wird, während dessen das Laserlicht (4) eine vordefinierte Augenöffnungsfläche (6) überstreicht, wobei während des Zeitbereichs eine Pulsgruppe von mehreren Laserpulsen aussendbar ist, und wobei das Energiekriterium einen Maximalwert für die Anzahl von Laserpulsen der Pulsgruppe und/oder für einen Abstand zweier Pulsgruppen aufweist.

6. Verfahren nach einem der vorhergehenden Ansprüche, **dadurch gekennzeichnet, dass** die Pulsparameter (100) einen Pulszeitpunkt und/oder eine Pulsenergie und/oder eine Pulslänge und/oder eine Pulsleistung und/oder einen Pulsaustrittswinkel umfassen.

7. Computerprogrammprodukt mit auf einem maschinenlesbaren Speichermedium gespeicherten Programmcode zur Durchführung des Verfahrens nach einem der vorhergehenden Ansprüche, wenn das Computerprogrammprodukt auf einer Rechenvorrichtung abläuft.

8. LiDAR-System (1) zum Aussenden von Laserlicht (4), umfassend
• eine Aussendeeinheit (2) zum Aussenden von Laserpulsen anhand von Pulsparametern, und
• ein Steuergerät (3) zum Ansteuern der Aussendeeinheit (2),
• wobei das Steuergerät (3) eingerichtet ist, ein Verfahren gemäß einem der Ansprüche 1 bis 6 durchzuführen.

## Claims

1. Method for operating a LiDAR system by transmitting laser light (4) in the form of laser pulses, comprising the steps of
• planning a laser pulse on the basis of pulse parameters (100),
• computing whether laser pulses that have been transmitted within a predefined past time interval meet a predefined energy criterion together with the planned pulse, the computation being carried out in real time, and
• transmitting the planned laser pulse by means of a transmission unit (2) if the energy criterion is met, and not transmitting the planned laser pulse or reducing a power of the laser pulse if the energy criterion is not met, wherein a predefined pulse pattern is transmitted repeatedly and wherein the energy criterion comprises a maximum value of an emission energy of the entire pulse pattern or wherein the energy criterion comprises a maximum value of a permitted pulse duration and/or pulse energy of the planned laser pulse and/or a minimum value of an interval between two laser pulses.

2. Method according to Claim 1, **characterized in that** the energy criterion is a maximum energy of the laser light that does not lead to permanent damage to a human eye.

3. Method according to either of the preceding claims, **characterized in that** all of the laser pulses are discrete laser pulses, in particular with a predefined minimum interval.

4. Method according to one of the preceding claims, **characterized in that** the laser light is transmitted in a scanning manner at a changing solid angle, wherein the solid angle is discretized into a grid of discrete pixels (5), wherein for each pixel (5) a light output of each laser pulse that hits the pixel (5) is integrated, and wherein the meeting of the energy criterion is checked for each pixel (5).

5. Method according to Claim 4, **characterized in that** a time range during which the laser light (4) sweeps over a predefined eye opening area (6) is determined, wherein a pulse group of multiple laser pulses is able to be transmitted during the time range, and wherein the energy criterion has a maximum value for the number of laser pulses in the pulse group and/or for an interval between two pulse groups.

6. Method according to one of the preceding claims, **characterized in that** the pulse parameters (100) comprise a pulse time and/or a pulse energy and/or a pulse length and/or a pulse power and/or a pulse exit angle.

7. Computer program product having program code, which is stored on a machine-readable storage medium, for carrying out the method according to one of the preceding claims when the computer program product runs on a computing apparatus.

8. LiDAR system (1) for transmitting laser light (4), comprising
• a transmission unit (2) for transmitting laser pulses on the basis of pulse parameters, and
• a control unit (3) for driving the transmission unit (2),
• the control unit (3) being designed to carry out a method according to one of Claims 1 to 6.

## Revendications

1. Procédé pour le fonctionnement d'un système LiDAR par émission de lumière laser (4) sous la forme d'impulsions laser, comprenant les étapes consistant à
• planifier une impulsion laser à l'aide de paramètres d'impulsion (100),
• calculer si les impulsions laser qui ont été émises dans un intervalle de temps précédent prédéfini satisfont, avec l'impulsion planifiée, un critère d'énergie prédéfini, le calcul étant effectué en temps réel et
• émettre l'impulsion laser planifiée au moyen d'une unité d'émission (2) lorsque le critère d'énergie est satisfait et ne pas émettre l'impulsion laser planifiée ou réduire une puissance de l'impulsion laser lorsque le critère d'énergie n'est pas satisfait, un motif d'impulsion prédéfini étant émis de manière répétitive et le critère d'énergie comprenant une valeur maximale d'une énergie d'émission de la totalité du motif d'impulsion entier ou le critère d'énergie comprenant une valeur maximale d'une durée d'impulsion autorisée et/ou une énergie d'impulsion de l'impulsion laser planifiée et/ou une valeur minimale d'une distance entre deux impulsions laser.

2. Procédé selon la revendication 1, **caractérisé en ce que** le critère d'énergie représente une énergie maximale de la lumière laser qui n'entraîne pas de dommages persistants dans un œil humain.

3. Procédé selon l'une des revendications précédentes, **caractérisé en ce que** la totalité des impulsions laser sont des impulsions laser discrètes, présentant notamment un espacement minimal prédéfini.

4. Procédé selon l'une des revendications précédentes, **caractérisé en ce que** la lumière laser est émise par balayage selon un angle solide variable, l'angle solide étant discrétisé en une grille de pixels discrets (5), une puissance lumineuse de chaque impulsion laser incidente sur le pixel (5) étant intégrée pour chaque pixel (5), et le fait que le critère d'énergie est satisfait étant vérifié pour chaque pixel (5).

5. Procédé selon la revendication 4, **caractérisé en ce qu'**une plage de temps est déterminée, pendant laquelle la lumière laser (4) balaye une surface d'ouverture d'oeil prédéfinie (6), un groupe d'impulsions de plusieurs impulsions laser pouvant être émis au cours de la plage de temps, et le critère d'énergie présentant une valeur maximale pour le nombre d'impulsions laser du groupe d'impulsions et/ou pour une distance entre deux groupes d'impulsions.

6. Procédé selon l'une des revendications précédentes, **caractérisé en ce que** les paramètres d'impulsion (100) comprennent un instant d'impulsion et/ou une énergie d'impulsion et/ou une longueur d'impulsion et/ou une puissance d'impulsion et/ou un angle de sortie d'impulsion.

7. Produit de programme d'ordinateur comportant un code de programme stocké sur un support de stockage lisible par machine pour la mise en œuvre du procédé selon l'une des revendications précédentes, lorsque le produit de programme d'ordinateur s'exécute sur un dispositif informatique.

8. Système LiDAR (1) destiné à émettre une lumière laser (4), comprenant
• une unité d'émission (2) pour émettre des impulsions laser sur la base de paramètres d'impulsion, et
• un appareil de commande (3) pour commander l'unité d'émission (2),
• l'appareil de commande (3) étant conçu pour mettre en œuvre un procédé selon l'une des revendications 1 à 6.
